(19) Europäisches Patentamt
European Patent Office
Office européen des brevets

(11) EP 0 919 221 A1

(12) DEMANDE DE BREVET EUROPEEN

(43) Date de publication:
**02.06.1999 Bulletin 1999/22**

(51) Int Cl.[6]: **A61K 7/13**

(21) Numéro de dépôt: **98402588.2**

(22) Date de dépôt: **19.10.1998**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **07.11.1997 FR 9714051**

(71) Demandeur: **L'OREAL**
**75008 Paris (FR)**

(72) Inventeur: **Maubru, Mireille**
**78400 Chatou (FR)**

(74) Mandataire: **Goulard, Sophie**
**L'OREAL-DPI**
**6 rue Bertrand Sincholle**
**92585 Clichy Cedex (FR)**

# (54) Composition pour la teinture d'oxydation des fibres kératiniques comprenant un 3,4-diamino pyrazole 5-substitué et un méta aminophénol halogéné et procédé de teinture

(57) La présente invention a pour objet une composition pour la teinture d'oxydation des fibres kératiniques, en particulier des fibres kératiniques humaines telles que les cheveux, comprenant au moins un 3,4-diamino pyrazole 5-substitué à titre de base d'oxydation, en association avec au moins un méta-aminophénol halogéné en ortho du phénol à titre de coupleur, ainsi que le procédé de teinture mettant en oeuvre cette composition avec un agent oxydant.

EP 0 919 221 A1

## Description

La présente invention a pour objet une composition pour la teinture d'oxydation des fibres kératiniques, en particulier des fibres kératiniques humaines telles que les cheveux, comprenant au moins un 3,4-diamino pyrazole 5-substitué à titre de base d'oxydation, en association avec au moins un méta-aminophénol halogéné en ortho du phénol à titre de coupleur, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être choisis notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet allemand DE 3 843 892, DE 4 234 887, DE 4 234 886, DE 4 234 885 ou DE 195 43 988 des compositions pour la teinture d'oxydation des fibres kératiniques contenant à titre de base d'oxydation des dérivés de pyrazole tels que des 4,5-diamino pyrazoles, des 3,4-diamino pyrazoles ou des 3,4,5-triamino pyrazoles, en association avec des coupleurs classiquement utilisés peur la teinture d'oxydation, tels que par exemple des métaphénylènediamines, des méta-aminophénols, des métadiphénols et des coupleurs hétérocycliques tels que par exemple des dérivés indoliques,. De telles compositions ne sont cependant pas entièrement satisfaisantes notamment du point de vue de la puissance des colorations obtenues.

Or, la demanderesse vient maintenant de découvrir qu'il est possible d'obtenir de nouvelles teintures puissantes et particulièrement résistantes aux diverses agressions que peuvent subir les cheveux, en associant, à titre de base d'oxydation, au moins un 3,4-diamino pyrazole 5-substitué de formule (I) définie ci-après et à titre de coupleur un méta-aminophénol halogéné en position ortho du phénol.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les 3,4-diamino pyrazoles 5-substitués de formule (I) suivante, et leurs sels d'addition avec un acide :

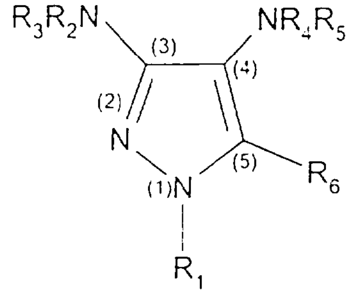

(I)

dans laquelle :

- $R_1$, $R_2$, $R_3$, $R_4$ et $R_5$, identiques ou différents, représentent un atome d'hydrogène ; un radical alkyle en $C_1$-$C_6$ linéaire ou ramifié ; un radical hydroxyalkyle en $C_2$-$C_4$ ; un radical aminoalkyle en $C_2$-$C_4$ ; un radical phényle ; un radical phényle substitué par un atome d'halogène ou un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, méthylènedioxy, hydroxy, hydroxyalkyle en $C_1$-$C_4$, amino, alkylamino en $C_1$-$C_4$ ; l'un au plus des radicaux $R_2$ à $R_5$ peut désigner un radical

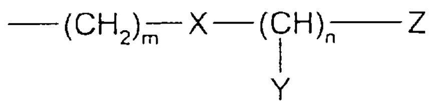

dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différents; désignent un atome d'hydrogène, un radical méthyle ou un radical éthyle ;

étant entendu que lorsque $R_2$ représente un atome d'hydrogène, alors $R_3$ peut représenter un radical amino ou alkylamino en $C_1$-$C_4$ ;

- $R_6$ représente un radical alkyle en $C_1$-$C_6$, linéaire ou ramifié ; un radical hydroxyalkyle en $C_1$-$C_4$ ; un radical aminoalkyle en $C_1$-$C_4$ ; un radical phényle ; un radical phényle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un hétérocycle choisi parmi le thiophène, le furane et la pyridine, ou encore un radical $-(CH_2)_p-O-(CH_2)_q-OR''$, dans lequel p et q sont des nombres entiers, identiques ou différents, compris entre 1 et 3 inclusivement et R'' représente un atome d'hydrogène ou un radical méthyle,

étant entendu que dans la formule (I) ci-dessus :

- au moins un des radicaux $R_4$ et $R_5$ représente un atome d'hydrogène,
- lorsque que $R_2$, respectivement $R_4$, représente un radical phényle substitué ou non, ou un radical benzyle ou un radical

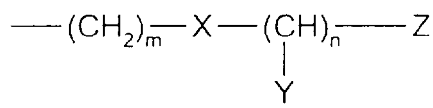

,

alors $R_3$, respectivement $R_5$, ne peut représenter aucun de ces trois radicaux,

- $R_1$ peut également représenter un reste hétérocyclique 2, 3 ou 4-pyridyle, 2 ou 3-thiényle, 2 ou 3-furyle éventuellement substitué par un radical méthyle.

- et au moins un coupleur choisi parmi les mita-aminophénols halogénés de formule (II) suivante, et leurs sels d'addition avec un acide :

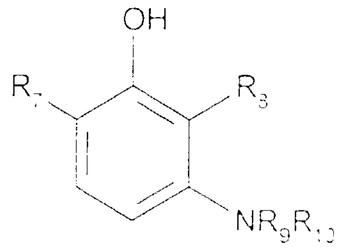

(II)

dans laquelle :

- $R_7$ et $R_8$, qui peuvent être identiques ou différents, représentent un atome d'hydrogène, un atome d'halogène tel que le chlore, le brome, l'iode ou le fluor, un radical alkyle en $C_1$-$C_4$, monohydroxyalkyle en $C_1$-$C_4$, polyhydroxyalkyle en $C_2$-$C_4$, alcoxy en $C_1$-$C_4$, monohydroxyalcoxy en $C_1$-$C_4$ ou polyhydroxyalcoxy en $C_2$-$C_4$ ;
- $R_9$ et $R_{10}$, qui peuvent être identiques ou différents, représentent un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;

étant entendu qu'au moins un des radicaux $R_7$ et $R_8$ représente un atome d'halogène.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes).

Parmi les radicaux alkyle en $C_1$-$C_4$ et alcoxy en $C_1$-$C_4$ des composés de formule (I) et (II) ci-dessus, on peut citer notamment les radicaux méthyle, éthyle, propyle, méthoxy et éthoxy.

Les 3,4-diamino pyrazoles 5-substitués sont des composés connus de l'art antérieur, qui peuvent être préparés par exemple selon les procédés de préparation décrits dans la demande de brevet français FR-A-2 748 274.

Parmi les 3,4-diamino pyrazoles 5-substitués de formule (I) utilisables à titre de base d'oxydation dans les compositions tinctoriales conformes à l'invention, on peut notamment citer :

- le 3,4-diamino-5-éthylpyrazole ;
- le 3,4-diamino-5-méthylpyrazole ;
- le 3,4-diamino-5-isopropylrazole ;
- le 3,4-diamino-5-tertio-butylpyrazole ;
- le 3,4-diamino-5-phénylpyrazole ;
- le 3,4-diamino-5-méthoxypyrazole ;
- le 3,4-diamino-5-(4'-méthoxyphényl)pyrazole ;
- le 3,4-diamino-5-(3'-méthoxyphényl)pyrazole ;
- le 3,4-diamino-5-(2'-méthoxyphényl)pyrazole ;
- le 3,4-diamino-5-(4'-méthylphényl)pyrazole ;
- le 3,4-diamino-5-(3'-méthylphényl)pyrazole ;
- le 3,4-diamino-5-(2'-chlorophényl)pyrazole ;
- le 3,4-diamino-5-(4'-chlorophényl)pyrazole ;
- le 3,4-diamino-5-(3'-trifluorométhylphényl)pyrazole ;
- le 3,4-diamino-1, 5-diméthylpyrazole ;
- le 3,4-diamino-5-éthyl-1-méthylpyrazole ;
- le 3,4-diamino-1-méthyl-5-tertio-butylpyrazole ;
- le 3,4-diamino-1-méthyl-5-phénylpyrazole ;
- le 3,4-diamino-1-méthyl-5-méthoxypyrazole ;
- le 3,4-diamino-1-méthyl-5-(4'-méthoxyphényl)pyrazole ;
- le 3,4-diamino-1-méthyl-5-(3'-méthoxyphényl)pyrazole ;
- le 3,4-diamino-1-méthyl-5-(2'-méthoxyphényl)pyrazole ;
- le 3,4-diamino-1-méthyl-5-(4'-méthylphényl)pyrazole ;
- le 3,4-diamino-1-méthyl-5-(3'-méthylphényl)pyrazole ;
- le 3,4-diamino-5-(2'-chlorophényl)-1-méthylpyrazole ;
- le 3,4-diamino-5-(4'-chlorophényl)-1-méthylpyrazole ;
- le 3,4-diamino-5-(3'-trifluorométhylphényl)-1-méthylpyrazole ;

- le 3,4-diamino-1-éthyl-5-méthylpyrazole ;
- le 3,4-diamino-1, 5-diéthylpyrazole ;
- le 3,4-diamino-1-éthyl-5-tertio-butylpyrazole ;
- le 3,4-diamino-1-éthyl-5-phénylpyrazole ;
- le 3,4-diamino-1-éthyl-5-méthoxypyrazole ;
- le 3,4-diamino-1-éthyl-5-(4'-méthoxyphényl)pyrazole ;
- le 3,4-diamino-1-éthy-5-(3'-méthoxyphényl)pyrazole ;
- le 3,4-diamino-1-éthyl-5-(2'-méthoxyphényl)pyrazole ;
- le 3,4-diamino-1-éthyl-5-(4'-méthylphényl)pyrazole ;
- le 3,4-diamino-1-éthyl-5-(3'-méthylphényl)pyrazole ;
- le 3,4-diamino-5-(2'-chlorophényl)-1-éthylpyrazole ;
- le 3,4-diamino-5-(4'-chlorophényl)-1-éthylpyrazole ;
- le 3,4-diamino-1-éthyl-5-(3'-trifluorométhylphényl)pyrazole ;
- le 3,4-diamino-1-isopropyl-5-méthylpyrazole ;
- le 3,4-diamino-5-éthyl-1-isopropylpyrazole ;
- le 3,4-diamino-1-isopropyl-5-tertio-butylpyrazole ;
- le 3,4-diamino-1-isopropyl-5-phényl-pyrazole ;
- le 3,4-diamino-1-isopropyl-5-méthoxypyrazole ;
- le 3,4-diamino-1-isopropyl-5-(4'-méthoxyphényl)pyrazole ;
- le 3,4-diamino-1-isopropyl-5-(3'-méthoxyphényl)pyrazole ;
- le 3,4-diamino-1-isopropyl-5-(2'-méthoxyphényl)pyrazole ;
- le 3,4-diamino-1-isopropyl-5-(4'-méthylphényl)pyrazole ;
- le 3,4-diamino-1-isopropyl-5-(3'-méthylphényl)pyrazole ;
- le 3,4-diamino-5-(2'-chlorophényl)-1-isopropylpyrazole ;
- le 3,4-diamino-5-(4'-chlorophényl)-1-isopropylpyrazole ;
- le 3,4-diamino-1-isopropyl-5-(3'-trifluorométhylphényl)pyrazole ;
- le 3,4-diamino-5-méthyl-1-propylpyrazole ;
- le 3,4-diamino-5-éthyl-1-propylpyrazole ;
- le 3,4-diamino-1-propyl-5-tertio-butylpyrazole ;
- le 3,4-diamino-5-phényl-1-propylpyrazole ;
- le 3,4-diamino-5-méthoxy-1-propylpyrazole ;
- le 3,4-diamino-5-(4'-méthoxyphényl)-1-propylpyrazole ;
- le 3,4-diamino-5-(3'-méthoxyphényl)-1-propylpyrazole ;
- le 3,4-diamino-5-(2'-méthoxyphényl)-1-propylpyrazole ;
- le 3,4-diamino-5-(4'-méthylphényl)-1-propylpyrazole ;
- le 3,4-diamine-5-(3'-méthylphényl)-1-propylpyrazole ;
- le 3,4-diamino-5-(2'-chlorophényl)-1-propylpyrazole ;
- le 3,4-diamino-5-(4'-chlorophényl)-1-propylpyrazole ;
- le 3,4-diamino-1-propyl-5-(3'-trifluorométhylphényl)pyrazole;
- le 1-benzyl-3,4-diamino-5-méthylpyrazole ;
- le 1-benzyl-3,4-diamino-5-éthylpyrazole ;
- le 1-benzyl-3,4-diamino-5-tertio-butylpyrazole ;
- le 1-benzyl-3,4-diamino-5-phénylpyrazole ;
- le 1-benzyl-3,4-diamino-5-méthoxypyrazole ;
- le 1-benzyl-3,4-diamino-5-(4'-méthoxyphényl)pyrazole ;
- le 1-benzyl-3,4-diamino-5-(3'-méthoxyphényl)pyrazole ;
- le 1-benzyl-3,4-diamino-5-(2'-méthoxyphényl)pyrazole ;
- le 1-benzyl-3,4-diamino-5-(4'-méthylphényl)pyrazole ;
- le 1-benzyl-3,4-diamino-5-(3'-méthylphényl)pyrazole ;
- le 1-benzyl-3,4-diamine-5-(2'-chlorophényl)pyrazole ;
- le 1-benzyl-3,4-diamino-5-(4'-chlorophényl)pyrazole ;
- le 1-benzyl-3,4-diamino-5-(3'-trifluorométhylphényl)pyrazole ;
- le 1-[4'-chlorobenzyl]-3,4-diamino-5-méthylpyrazole ;
- le 1-[4'-chlorobenzyl]-3,4-diamino-5-éthylpyrazole ;
- le 1-[4'-chlorobenzyl]-3,4-diamino-5-tertio-butylpyrazole ;
- le 1-[4'-chlorobenzyl]-3,4-diamino-5-phénylpyrazole ;
- le 1-[4'-chlorobenzyl]-3,4-diamino-5-méthoxypyrazole ;
- le 1-[4'-chlorobenzyl]-3,4-diamino-5-(4'-méthoxyphényl)pyrazole ;

- le 1-[4'-chlorobenzyl]-3,4-diamino-5-(3'-méthoxyphényl)pyrazole ;
- le 1-[4'-chlorobenzyl]-3,4-diamino-5-(2'-méthoxyphényl)pyrazole ;
- le 1-[4'-chlorobenzyl]-3,4-diamino-5-(4'-méthylphényl)pyrazole ;
- le 1-[4'-chlorobenzyl]-3,4-diamino-5-(3'-méthylphényl)pyrazole ;
- le 1-[4'-chlorobenzyl]-3,4-diamino-5-(2'-chlorophényl)pyrazole ;
- le 1-[4'-chlorobenzyl]-3,4-diamino-5-(4'-chlorophényl)pyrazole ;
- le-1-[4'-chlorobenzyl]-3,4-diamino-5-(3'-trifluorométhylphényl)pyrazole ;
- le 3,4-diamino-5-hydroxyméthyl-1-méthylpyrazole ;
- le 3,4-diamino-5-hydroxyméthyl-1-éthylpyrazole ;
- le 3,4-diamino-5-hydroxyméthyl-1-isopropylpyrazole ;
- le 3,4-diamino-5-hydroxyméthyl-1-propylpyrazole ;
- le 1-benzyl-3,4-diamino-5-hydroxyméthylpyrazole ;
- le 1-[4'-chlorobenzyl]-3,4-diamino-5-hydroxyméthylpyrazole ;
- le 5-aminométhyl-3,4-diamino-1-méthylpyrazole ;
- le 5-aminométhyl-3,4-diamino-1-éthylpyrazole ;
- le 5-aminométhyl-3,4-diamino-1-isopropylpyrazole ;
- le 5-aminométhyl-3,4-diamino-1-propylpyrazole ;
- le 5-aminométhyl-1-benzyl-3,4-diaminopyrazole ;
- le 5-aminométhyl-1-[4'-chlorobenzyl]-3,4-diaminopyrazole ;
- le 3,4-diamino-5-hydroxyméthylpyrazole ;
- le 3,4-diamino-5-[β-hydroxyéthylamino]-1-méthylpyrazole ;
- le 3,4-diamino-5-[β-hydroxyéthylamino]-1-éthylpyrazole ;
- le 3,4-diamino-5-[β-hydroxyéthylamino]-1-isopropylpyrazole ;
- le 3,4-diamino-5-[β-hydroxyéthylamino]-1-propylpyrazole ;
- le 3,4-diamino-5-[β-hydroxyéthylamino]pyrazole ;
- le 1-benzyl-3,4-diamino-5-[β-hydroxyéthylamino]pyrazole ;
- le 1-[4'-chlorobenzyl]-3,4-diamino-5-[β-hydroxyéthylamino]pyrazole ;

et leurs sels d'addition avec un acide.

Parmi ces 3,4-diamino pyrazoles 5-substitués, on préfère plus particulièrement :

- le 3,4-diamino-5-méthylpyrazole ;
- le 3,4-diamino-5-éthylpyrazole ;
- le 3,4-diamino-5-isopropylpyrazole ;
- le 3,4-diamino-5-tertio-butylpyrazole ;
- le 3,4-diamino-5-phénylpyrazole ;
- le 3,4-diamino-5-(4'-méthoxyphényl)pyrazole ;
- le 3,4-diamino-5-(3'-méthoxyphényl)pyrazole ;
- le 3,4-diamino-5-(2'-méthoxyphényl)pyrazole ;
- le 3,4-diamino-5-(4'-méthylphényl)pyrazole ;
- le 3,4-diamino-5-(3'-méthylphényl)pyrazole ;
- le 5-(2'-chlorophényl)-3,4-diaminopyrazole ;
- le 5-(4'-chlorophényl)-3,4-diaminopyrazole ;
- le 3,4-diamino-1, 5-diméthylpyrazole ;
- le 3,4-diamino-5-éthyl-1-méthylpyrazole ;
- le 3,4-diamino-5-isopropyl-1-méthylpyrazole ;
- le 3,4-diamino-1-méthyl-5-tertio-butylpyrazole ;
- le 3,4-diamino-1-méthyl-5-phénylpyrazole ;
- le 3,4-diamino-1-méthyl-5-méthoxypyrazole ;
- le 3,4-diamino-1-méthyl-5-(4'-méthoxyphényl)pyrazole ;
- le 3,4-diamino-1-méthyl-5-(3'-méthoxyphényl)pyrazole ;
- le 3,4-diamino-1-méthyl-5-(2'-méthoxyphényl)pyrazole ;
- le 3,4-diamino-1-méthyl-5-(4'-méthylphényl)pyrazole ;
- le 3,4-diamino-1-méthyl-5-(3'-méthylphényl)pyrazole ;
- le 5-(2'-chlorophényl)-3,4-diamino-1-méthylpyrazole ;
- le 5-(4'-chlorophényl)-3,4-diamino-1-méthylpyrazole ;

et leurs sels d'addition avec un acide.

Parmi les méta-aminophénols halogénés de formule (II) utilisables à titre de coupleur dans les compositions tinctoriales conformes à l'invention, on peut plus particulièrement citer le 3-amino 6-chloro phénol, le 3-amino 6-bromo phénol, le 3-(β-aminoéthyl)amino 6-chlore phénol, le 3-(β-hydroxyéthyl)amino 6-chloro phénol, le 3-amino 2-chloro 6-méthyl phénol, et leurs sels d'addition avec un acide.

Le ou les 3,4-diamino pyrazoles 5-substitués de formule (I) et/ou le ou les sels d'addition avec un acide correspondants représentent de préférence de 0,0005 à 12 % en poids environ du poids total de la composition tinctoriale, et encore plus préférentiellement de 0,005 à 6 % en poids environ de ce poids.

Le ou les méta-aminophénols halogénés de formule (II) conformes à l'invention et/ou le ou les sels d'addition avec un acide correspondants, représentent de préférence de 0,0001 à 5 % en poids environ du poids total de la composition tinctoriale, et encore plus préférentiellement de 0,005 à 3 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Les compositions tinctoriales conformes à l'invention peuvent contenir d'autres bases d'oxydation classiquement utilisées pour la teinture d'oxydation, différentes des 3,4-diamino pyrazoles 5-substitués de formule (I) et/ou d'autres coupleurs classiquement utilisés pour la teinture d'oxydation, différents des méta-aminophénols halogénés de formule (II), et/ou des colorants directs notamment pour modifier les nuances ou les enrichir en reflets.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthane et l'isopropanol ; le glycérol ; les glycols et éthers de glycols comme le 2-butoxyélhanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante :

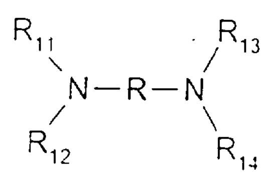

(III)

dans laquelle R est un reste propylène éventuellement substitué par un groupement hydroxyle ou un radical alkyle en $C_1$-$C_4$ ; $R_{11}$, $R_{12}$, $R_{13}$ et $R_{14}$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme

de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 60 minutes environ, de préférence 5 à 40 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates, percarbonates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**EXEMPLES DE TEINTURE COMPARATIFS**

On a préparé les compositions tinctoriales, conformes à l'invention, suivantes (teneurs en grammes) :

| EXEMPLE | 1 (*) | 2 | 3 | 4 |
|---|---|---|---|---|
| Dichlorhydrate de 3,4-diamino 5-méthyl pyrazole (Base d'oxydation) | 0,555 | 0,555 | 0,555 | 0,555 |
| 3-amino phénol (coupleur ne faisant pas partie de l'invention) | 0,327 | - | - | - |
| 3-amino 6-chloro phénol (coupleur conforme à l'invention) | - | 0,431 | - | - |
| 3-(β-aminoéthyl)amino 6-chloro phénol (coupleur conforme à l'invention) | - | - | 0,560 | - |
| 3-amino 2-chloro 6-méthyl phénol (coupleur conforme à l'invention) | - | - | - | 0,473 |

(*) : exemple ne faisant pas partie de l'invention

(suite)

| EXEMPLE | 1 (*) | 2 | 3 | 4 |
|---|---|---|---|---|
| Support de teinture commun | (**) | (**) | (**) | (**) |
| Eau déminéralisée q.s.p. | 100 g | 100 g | 100 g | 100 g |

(*) : exemple ne faisant pas partie de l'invention

(**) support de teinture commun :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 par la société AKZO 7,0 g
- Laurylamino succinamate de diéthylaminopropyle, sel de sodium, à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10 g

Il est important de noter que chacune des compositions tinctoriales 1'à 4 ci-dessus contient la même quantité molaire de coupleur, à savoir $3.10^{-3}$ mole.

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris permanentés à 90 % de blancs. Les mèches de cheveux ont ensuite été rincées, lavées avec un shampooing standard puis séchées.

La couleur des mèches a été évaluée avant et après la coloration, dans le système MUNSELL, au moyen d'un colorimètre CM 2002 MINOLTA, de façon à déterminer la puissance des colorations obtenues avec chacune des compositions décrites ci-dessus.

La différence entre la couleur de la mèche avant la teinture et la couleur de la mèche après la teinture a été calculée en appliquant la formule de NICKERSON :

$$\Delta E = 0,4\ Co\Delta H + 6\Delta V + 3\ \Delta C$$

telle que décrite par exemple dans "Couleur, Industrie et Technique" ; pages 14-17 ; vol. n° 5 ; 1978.

Dans cette formule, $\Delta E$ représente la différence de couleur entre deux mèches, $\Delta H$, $\Delta V$ et $\Delta C$ représentent la variation en valeur absolue des paramètres H, V et C et Co représente la pureté de la mèche par rapport à laquelle on désire évaluer la différence de couleur.

La puissance de la coloration ($\Delta E$) est d'autant plus importante que le chiffre indiqué est élevé.

Les résultats sont donnés dans le tableau I ci-dessous :

Tableau I

| EXEMPLE | Couleur des cheveux avant la teinture | Couleur des cheveux après la teinture | Puissance de la coloration | | | |
|---|---|---|---|---|---|---|
| | | | ΔH | ΔV | ΔC | ΔE |
| 1 (*) | 4,0 Y 5,1 / 1,5 | 3,6 R 3,2 / 3,4 | 20,4 | 1,9 | 1,9 | **29,3** |
| 2 | 4,0 Y 5,1 / 1,5 | 6,4 RP 2,5 / 3,4 | 27,6 | 2,6 | 1,9 | **37,9** |
| 3 | 4,0 Y 5,1 / 1,5 | 6,8 RP 2,6 / 2,7 | 27,2 | 2,5 | 1,2 | **34,9** |
| 4 | 4,0 Y 5,1 / 1,5 | 0,1 R 2,6 / 4,5 | 23,9 | 2,5 | 3,0 | **38,3** |

(*) : Exemple ne faisant pas partie de l'invention.

Ces résultats montrent que la coloration obtenue en mettant en oeuvre la composition tinctoriale de l'exemple 1 ne faisant pas partie de l'invention car contenant l'association d'un 3,4-diamino pyrazole 5-substitué et d'un méta-aminophénol non halogéné, conduit à une coloration nettement moins puissante que les colorations obtenues en mettant en oeuvre les compositions des exemples 2 à 4, faisant toutes partie de l'invention car contenant l'association d'un 3,4-diamino pyrazole 5-substitué et d'un méta-aminophénol halogéné en ortho du phénol.

**Revendications**

**1.** Composition pour la teinture d'oxydation des fibres kératiniques humaines et en particulier des fibres kératiniques humaines telles que les cheveux, caractérisée par le fait qu'elle comprend, dans un milieu approprié pour la teinture :

- au moins une base d'oxydation choisie parmi les 3,4-diamino pyrazoles 5-substitués de formule (I) suivante, et leurs sels d'addition avec un acide :

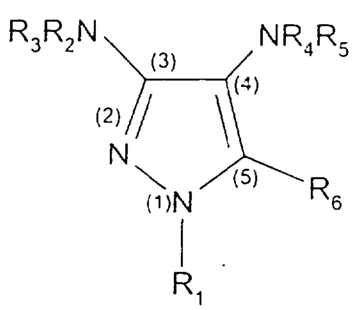

(I)

dans laquelle :

- $R_1$, $R_2$, $R_3$, $R_4$ et $R_5$, identiques ou différents, représentent un atome d'hydrogène ; un radical alkyle en $C_1$-$C_6$ linéaire ou ramifié ; un radical hydroxyalkyle en $C_2$-$C_4$ ; un radical aminoalkyle en $C_2$-$C_4$ ; un radical phényle ; un radical phényle substitué par un atome d'halogène ou un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, méthylènedioxy, hydroxy, hydroxyalkyle en $C_1$-$C_4$, amino, alkylamino en $C_1$-$C_4$ ; l'un au plus des radicaux $R_2$ à $R_5$ peut désigner un radical

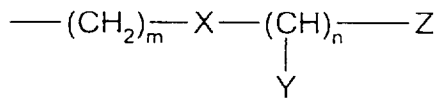

dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différents, désignent un atome d'hydrogène, un radical méthyle ou un radical éthyle ;

étant entendu que lorsque $R_2$ représente un atome d'hydrogène, alors $R_3$ peut représenter un radical amino ou alkylamino en $C_1$-$C_4$ ;

- $R_6$ représente un radical alkyle en $C_1$-$C_6$, linéaire ou ramifié ; un radical hydroxyalkyle en $C_1$-$C_4$ ; un radical aminoalkyle en $C_1$-$C_4$ ; un radical phényle ; un radical phényle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un radical benzyle ; un radical benzyle substitué par un atome d'halogène ou par un radical alkyle en $C_1$-$C_4$, alcoxy en $C_1$-$C_4$, nitro, trifluorométhyle, amino ou alkylamino en $C_1$-$C_4$ ; un hétérocycle choisi parmi le thiophène, le furane et la pyridine, ou encore un radical $-(CH_2)_p-O-(CH_2)_q-OR''$, dans lequel p et q sont des nombres entiers, identiques ou différents, compris entre 1 et 3 inclusivement et R" représente un atome d'hydrogène ou un radical méthyle,

étant entendu que dans la formule (I) ci-dessus :

- au moins un des radicaux $R_4$ et $R_5$ représente un atome d'hydrogène,
- lorsque que $R_2$, respectivement $R_4$, représente un radical phényle substitué ou non, ou un radical benzyle ou un radical

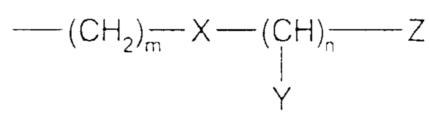

,

alors $R_3$, respectivement $R_5$, ne peut représenter aucun de ces trois radicaux,

- $R_1$ peut également représenter un reste hétérocyclique 2, 3 ou 4-pyridyle, 2 ou 3-thiényle, 2 ou 3-furyle éventuellement substitué par un radical méthyle.

- et au moins un coupleur choisi parmi les méta-aminophénols halogénés de formule (II) suivante, et leurs sels d'addition avec un acide :

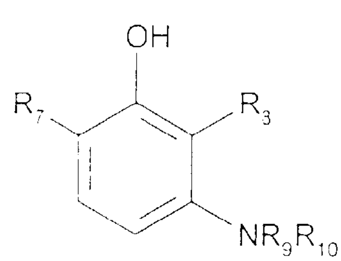

(II)

dans laquelle :

- $R_7$ et $R_8$, qui peuvent être identiques ou différents, représentent un atome d'hydrogène, un atome d'halogène tel que le chlore, le brome, l'iode ou le fluor, un radical alkyle en $C_1$-$C_4$, monohydroxyalkyle en $C_1$-$C_4$, polyhydroxyalkyle en $C_2$-$C_4$, alcoxy en $C_1$-$C_4$, monohydroxyalcoxy en $C_1$-$C_4$ ou polyhydroxyalcoxy en $C_2$-$C_4$ ;
- $R_9$ et $R_{10}$, qui peuvent être identiques ou différents, représentent un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;

étant entendu qu'au moins un des radicaux $R_7$ et $R_8$ représente un atome d'halogène.

**2.** Composition selon la revendication 1, caractérisée par le fait que le ou les 3,4-diamino pyrazoles 5-substitués de formule (I) sont choisis parmi :

- le 3,4-diamino-5-éthylpyrazole ;
- le 3,4-diamino-5-méthylpyrazole ;
- le 3,4-diamino-5-isopropylrazole ;
- le 3,4-diamino-5-tertio-butylpyrazole ;
- le 3,4-diamino-5-phénylpyrazole ;
- le 3,4-diamino-5-méthoxypyrazole ;
- le 3,4-diamino-5-(4'-méthoxyphényl)pyrazole ;
- le 3,4-diamino-5-(3'-méthoxyphényl)pyrazole ;
- le 3,4-diamino-5-(2'-méthoxyphényl)pyrazole ;
- le 3,4-diamino-5-(4'-méthylphényl)pyrazole ;
- le 3,4-diamino-5-(3'-méthylphényl)pyrazole ;
- le 3,4-diamino-5-(2'-chlorophényl)pyrazole ;
- le 3,4-diamino-5-(4'-chlorophényl)pyrazole ;
- le 3,4-diamino-5-(3'-trifluorométhylphényl)pyrazole ;
- le 3,4-diamino-1, 5-diméthylpyrazole ;
- le 3,4-diamino-5-éthyl-1-méthylpyrazole ;
- le 3,4-diamino-1-méthyl-5-tertio-butylpyrazole ;
- le 3,4-diamino-1-méthyl-5-phénylpyrazole ;
- le 3,4-diamino-1-méthyl-5-méthoxypyrazole ;
- le 3,4-diamino-1-méthyl-5-(4'-méthoxyphényl)pyrazole ;
- le 3,4-diamino-1-mélhyl-5-(3'-méthoxyphényl)pyrazole ;
- le 3,4-diamino-1-méthyl-5-(2'-méthoxyphényl)pyrazole ;
- le 3,4-diamino-1-méthyl-5-(4'-méthylphényl)pyrazole ;
- le 3,4-diamino-1-méthyl-5-(3'-méthylphényl)pyrazole ;
- le 3,4-diamino-5-(2'-chlorophényl)-1-méthylpyrazole ;
- le 3,4-diamino-5-(4'-chlorophényl)-1-méthylpyrazole ;
- le 3,4-diamino-5-(3'-trifluorométhylphényl)-1-méthylpyrazole ;
- le 3,4-diamino-1-éthyl-5-méthylpyrazole ;
- le 3,4-diamino-1,5-diéthylpyrazole ;
- le 3,4-diamino-1-éthyl-5-tertio-butylpyrazole ;
- le 3,4-diamino-1-éthyl-5-phénylpyrazole ;
- le 3,4-diamino-1-éthyl-5-méthoxypyrazole ;
- le 3,4-diamino-1-éthyl-5-(4'-méthoxyphényl)pyrazole ;
- le 3,4-diamino-1-éthy-5-(3'-méthoxyphényl)pyrazole ;
- le 3,4-diamino-1-éthyl-5-(2'-méthoxyphényl)pyrazole ;
- le 3,4-diamino-1-éthyl-5-(4'-méthylphényl)pyrazole ;
- le 3,4-diamino-1-éthyl-5-(3'-méthylphényl)pyrazole ;
- le 3,4-diamino-5-(2'-chlorophényl)-1-éthylpyrazole ;
- le 3,4-diamino-5-(4'-chlorophényl)-1-éthylpyrazole ;
- le 3,4-diamino-1-éthyl-5-(3'-trifluorométhylphényl)pyrazole ;
- le 3,4-diamino-1-isopropyl-5-méthylpyrazole ;
- le 3,4-diamino-5-éthyl-1-isopropylpyrazole ;
- le 3,4-diamino-1-isopropyl-5-tertio-butylpyrazole ;
- le 3,4-diamino-1-isopropyl-5-phényl-pyrazole ;
- le 3,4-diamino-1-isopropyl-5-méthoxypyrazole ;
- le 3,4-diamino-1-isopropyl-5-(4'-méthoxyphényl)pyrazole ;

- le 3,4-diamino-1-isopropyl-5-(3'-méthoxyphényl)pyrazole ;
- le 3,4-diamino-1-isopropyl-5-(2'-méthoxyphényl)pyrazole ;
- le 3,4-diamino-1-isopropyl-5-(4'-méthylphényl)pyrazole ;
- le 3,4-diamino-1-isopropyl-5-(3'-méthylphényl)pyrazole ;
- le 3,4-diamino-5-(2'-chlorophényl)-1-isopropylpyrazole ;
- le 3,4-diamino-5-(4'-chlorophényl)-1-isopropylpyrazole ;
- le 3,4-diamino-1-isopropyl-5-(3'-trifluorométhylphényl)pyrazole ;
- le 3,4-diamino-5-méthyl-1-propylpyrazole ;
- le 3,4-diamino-5-éthyl-1-propylpyrazole ;
- le 3,4-diamino-1-propyl-5-tertio-butylpyrazole ;
- le 3,4-diamino-5-phényl-1-propylpyrazole ;
- le 3,4-diamino-5-méthoxy-1-propylpyrazole ;
- le 3,4-diamino-5-(4'-méthoxyphényl)-1-propylpyrazole ;
- le 3,4-diamino-5-(3'-méthoxyphényl)-1-propylpyrazole ;
- le 3,4-diamino-5-(2'-méthoxyphényl)-1-propylpyrazole ;
- le 3,4-diamino-5-(4'-méthylphényl)-1-propylpyrazole ;
- le 3,4-diamino-5-(3'-méthylphényl)-1-propylpyrazole ;
- le 3,4-diamino-5-(2'-chlorophényl)-1-propylpyrazole ;
- le 3,4-diamino-5-(4'-chlorophényl)-1-propylpyrazole ;
- le 3,4-diamino-1-propyl-5-(3'-trifluorométhylphényl)pyrazole;
- le 1-benzyl-3,4-diamino-5-méthylpyrazole ;
- le 1-benzyl-3,4-diamino-5-éthylpyrazole ;
- le 1-benzyl-3,4-diamino-5-tertio-butylpyrazole ;
- le 1-benzyl-3,4-diamino-5-phénylpyrazole ;
- le 1-benzyl-3,4-diamino-5-méthoxypyrazole ;
- le 1-benzyl-3,4-diamino-5-(4'-méthoxyphényl)pyrazole ;
- le 1-benzyl-3,4-diamino-5-(3'-méthoxyphényl)pyrazole ;
- le 1-benzyl-3,4-diamino-5-(2'-méthoxyphényl)pyrazole ;
- le 1-benzyl-3,4-diamino-5-(4'-méthylphényl)pyrazole ;
- le 1-benzyl-3,4-diamino-5-(3'-méthylphényl)pyrazole ;
- le 1-benzyl-3,4-diamino-5-(2'-chlorophényl)pyrazole ;
- le 1-benzyl-3,4-diamino-5-(4'-chlorophényl)pyrazole ;
- le 1-benzyl-3,4-diamino-5-(3'-trifluorométhylphényl)pyrazole ;
- le 1-[4'-chlorobenzyl]-3,4-diamino-5-méthylpyrazole ;
- le 1-[4'-chlorobenzyl]-3,4-diamino-5-éthylpyrazole ;
- le 1-[4'-chlorobenzyl]-3,4-diamino-5-tertio-butylpyrazole ;
- le 1-[4'-chlorobenzyl]-3,4-diamino-5-phénylpyrazole ;
- le 1-[4'-chlorobenzyl]-3,4-diamino-5-méthoxypyrazole ;
- le 1-[4'-chlorobenzyl]-3,4-diamino-5-(4'-méthoxyphényl)pyrazole ;
- le 1-[4'-chlorobenzyl]-3,4-diamino-5-(3'-méthoxyphényl)pyrazole ;
- le 1-[4'-chlorobenzyl]-3,4-diamino-5-(2'-méthoxyphényl)pyrazole ;
- le 1-[4'-chlorobenzyl]-3,4-diamino-5-(4'-méthylphényl)pyrazole ;
- le 1-[4'-chlorobenzyl]-3,4-diamino-5-(3'-méthylphényl)pyrazole ;
- le 1-[4'-chlorobenzyl]-3,4-diamino-5-(2'-chlorophényl)pyrazole ;
- le 1-[4'-chlorobenzyl]-3,4-diamino-5-(4'-chlorophényl)pyrazole ;
- le 1-[4'-chlorobenzyl]-3,4-diamino-5-(3'-trifluorométhylphényl)pyrazole ;
- le 3,4-diamino-5-hydroxyméthyl-1-méthylpyrazole ;
- le 3,4-diamino-5-hydroxyméthyl-1-éthylpyrazole ;
- le 3,4-diamino-5-hydroxyméthyl-1-isopropylpyrazole ;
- le 3,4-diamino-5-hydroxyméthyl-1-propylpyrazole ;
- le 1-benzyl-3,4-diamino-5-hydroxyméthylpyrazole ;
- le 1-[4'-chlorobenzyl]-3,4-diamino-5-hydroxyméthylpyrazole ;
- le 5-aminométhyl-3,4-diamino-1-méthylpyrazole ;
- le 5-aminométhyl-3,4-diamino-1-éthylpyrazole ;
- le 5-aminométhyl-3,4-diamino-1-isopropylpyrazole ;
- le 5-anninométhyl-3,4-diamino-1-propylpyrazole ;
- le 5-aminométhyl-1-benzyl-3,4-diaminopyrazole ;
- le 5-aminométhyl-1-[4'-chlorobenzyl]-3,4-diaminopyrazole ;

- le 3,4-diamino-5-hydroxyméthylpyrazole ;
- le 3,4-diamino-5-[β-hydroxyéthylamino]-1-méthylpyrazole ;
- le 3,4-diamino-5-[β-hydroxyéthylamino]-1-éthylpyrazole ;
- le 3,4-diamino-5-[β-hydroxyéthylamino]-1-isopropylpyrazole ;
- le 3,4-diamino-5-[β-hydroxyéthylamino]-1-propylpyrazole ;
- le 3,4-diamino-5-[β-hydroxyéthylamino]pyrazole ;
- le 1-benzyl-3,4-diamino-5-[β-hydroxyéthylamino]pyrazole ;
- le 1-[4'-chlorobenzyl]-3,4-diamino-5-[β-hydroxyéthylamino]pyrazole ;

et leurs sels d'addition avec un acide.

**3.** Composition selon la revendication 2, caractérisée par le fait que le ou les 3,4-diamino pyrazoles 5-substitués de formule (I) sont choisis parmi :

- le 3,4-diamino-5-méthylpyrazole ;
- le 3,4-diamino-5-éthylpyrazole ;
- le 3,4-diamino-5-isopropylpyrazole ;
- le 3,4-diamino-5-tertio-butylpyrazole ;
- le 3,4-diamino-5-phénylpyrazole ;
- le 3,4-diamino-5-(4'-méthoxyphényl)pyrazole ;
- le 3,4-diamino-5-(3'-méthoxyphényl)pyrazole ;
- le 3,4-diamino-5-(2'-méthoxyphényl)pyrazole ;
- le 3,4-diamino-5-(4'-méthylphényl)pyrazole ;
- le 3,4-diamino-5-(3'-méthylphényl)pyrazole ;
- le 5-(2'-chlorophényl)-3,4-diaminopyrazole ;
- le 5-(4'-chlorophényl)-3,4-diaminopyrazole ;
- le 3,4-diamino-1, 5-diméthylpyrazole ;
- le 3,4-diamino-5-éthyl-1-méthylpyrazole ;
- le 3,4-diamino-5-isopropyl-1-méthylpyrazole ;
- le 3,4-diamino-1-méthyl-5-tertio-butylpyrazole ;
- le 3,4-diamino-1-méthyl-5-phénylpyrazole ;
- le 3,4-diamino-1-méthyl-5-méthoxypyrazole ;
- le 3,4-diamino-1-méthyl-5-(4'-méthoxyphényl)pyrazole ;
- le 3,4-diamino-1-méthyl-5-(3'-méthoxyphényl)pyrazole ;
- le 3,4-diamino-1-méthyl-5-(2'-méthoxyphényl)pyrazole ;
- le 3,4-diamino-1-méthyl-5-(4'-méthylphényl)pyrazole ;
- le 3,4-diamino-1-méthyl-5-(3'-méthylphényl)pyrazole ;
- le 5-(2'-chlorophényl)-3,4-diamino-1-méthylpyrazole ;
- le 5-(4'-chlorophényl)-3,4-diamino-1-méthylpyrazole ;

et leurs sels d'addition avec un acide.

**4.** Composition selon l'une quelconque des revendications précédentes, caractérisée par le fait que les méta-aminophénols halogénés de formule (II) sont choisis parmi le 3-amino 6-chloro phénol, le 3-amino 6-bromo phénol, le 3-(β-aminoéthyl)amino 6-chloro phénol, le 3-(β-hydroxyéthyl)amino 6-chloro phénol, le 3-amino 2-chloro 6-méthyl phénol, et leurs sels d'addition avec un acide.

**5.** Composition selon l'une quelconque des revendications précédentes, caractérisée par le fait que le ou les 3,4-diamino pyrazoles 5-substitués de formule (I) et/ou le ou les sels d'addition avec un acide correspondants représentent de 0,0005 à 12 % en poids du poids total de la composition tinctoriale.

**6.** Composition selon la revendication 5, caractérisée par le fait que le ou les 3,4-diamino pyrazoles 5-substitués de formule (I) et/ou le ou les sels d'addition avec un acide correspondants représentent de 0,005 à 6 % en poids du poids total de la composition tinctoriale.

**7.** Composition selon l'une quelconque des revendications précédentes, caractérisée par le fait que le ou les méta-aminophénols halogénés de formule (II) et/ou le ou les sels d'addition avec un acide correspondants représentent de 0,0001 à 5% en poids du poids total de la composition tinctoriale.

**8.** Composition selon la revendication 7, caractérisée par le fait que le ou les méta-aminophénols halogénés de formule (II) et/ou le ou les sels d'addition avec un acide correspondants représentent de 0,005 à 3 % en poids du poids total de la composition tinctoriale.

**9.** Composition selon l'une quelconque des revendications précédentes, caractérisée par le fait que les sels d'addition avec un acide sont choisis parmi les chlorhydrates, les bromhydrates, les sulfates et les tartrates, les lactates et les acétates.

**10.** Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**11.** Composition selon l'une quelconque des revendications précédentes, caractérisée par le fait qu'elle présente un pH compris entre 3 et 12.

**12.** Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

**13.** Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 1 à 12, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

**14.** Procédé selon la revendication 13, caractérisé par le fait que l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides.

**15.** Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante.

Office européen des brevets

# RAPPORT DE RECHERCHE EUROPEENNE

Numéro de la demande
EP 98 40 2588

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP 0 740 931 A (L'OREAL) 6 novembre 1996<br>* revendications 1,2,4,13 *<br>* page 6, ligne 57 - page 7, ligne 9 * | 1 | A61K7/13 |
| A | DE 44 22 603 A (WELLA) 4 janvier 1996<br>* revendications 1,4 * | 1 | |

DOMAINES TECHNIQUES RECHERCHES (Int.Cl.6)

A61K

Le présent rapport a été établi pour toutes les revendications

| Lieu de la recherche | Date d'achèvement de la recherche | Examinateur |
|---|---|---|
| LA HAYE | 15 janvier 1999 | Peeters, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 98 40 2588

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-01-1999

| Document brevet cité au rapport de recherche | Date de publication | Membre(s) de la famille de brevet(s) | Date de publication |
|---|---|---|---|
| EP 740931 A | 06-11-1996 | FR 2733749 A | 08-11-1996 |
| | | AT 156998 T | 15-09-1997 |
| | | CA 2217333 A | 07-11-1996 |
| | | DE 69600054 D | 25-09-1997 |
| | | DE 69600054 T | 15-01-1998 |
| | | ES 2109111 T | 01-01-1998 |
| | | WO 9634591 A | 07-11-1996 |
| | | JP 10506672 T | 30-06-1998 |
| | | PL 323132 A | 16-03-1998 |
| DE 4422603 A | 04-01-1996 | BR 9502946 A | 12-03-1996 |
| | | DE 59500205 D | 05-06-1997 |
| | | EP 0692245 A | 17-01-1996 |
| | | ES 2083941 T | 01-05-1996 |
| | | JP 8012540 A | 16-01-1996 |
| | | US 5718731 A | 17-02-1998 |

EPO FORM P0460

Pour tout renseignement concernant cette annexe : voir Journal Officiel de l'Office européen des brevets, No.12/82